# EUROPEAN PATENT SPECIFICATION

(11) **EP 2 672 964 B1**
(45) Date of publication and mention of the grant of the patent: **01.06.2016**
(21) Application number: 12708937.3
(22) Date of filing: 10.02.2012
(51) Int. Cl.: A61K 31/05, A61K 31/353, A61K 36/48, A61K 36/87, A61P 15/12

(54) **COMPOSITION FOR USE IN THE TREATMENT OF MENOPAUSE PROBLEMS/DISORDERS**
ZUSAMMENSETZUNG ZUR VERWENDUNG IN DER BEHANDLUNG VON BESCHWERDEN / ERKRANKUNGEN DER MENOPAUSE
COMPOSITION POUR L' UTILISATION DANS LE TRAITEMENT DES PROBLÈMES/TROUBLES DE LA MENOPAUSE

(30) Priority: 10.02.2011 IT MI20110198
(43) Date of publication of application: 18.12.2013
(73) Proprietor: Paladin Pharma S.p.A., 10126 Torino (IT)
(72) Inventor: SCAPAGNINI, Giovanni, 95131 Catania (IT); DAVINELLI, Sergio, I-86100 Campobasso (IT); VISENTIN, Manuela, I-10136 Torino (IT); RUFFINENGO, Edoardo, I-10126 Torino (IT)
(74) Representative: Coppo, Alessandro
(86) International application number: PCT/IB2012/050615
(87) International publication number: WO 2012/107905

(56) References cited:
- EP-A2- 1 656 942
- EP-A2- 2 014 282
- WO-A1-2009/114525
- WO-A2-01/70212
- US-A1- 2008 254 055
- LANGE SKOVGAARD G R ET AL: "Effect of a novel dietary supplement on skin aging in post-menopausal women", EUROPEAN JOURNAL OF CLINICAL NUTRITION, XX, XX, vol. 60, 1 January 2006 (2006-01-01), pages 1201-1206, XP008080469,
- Simply Natural: "Phyto Soya Night & Day @ Simply Natural", Simply Natural Health Supplement & Homebrew Specialists , 28 August 2008 (2008-08-28), XP002659774, Retrieved from the Internet: URL:http://web.archive.org/web/20080828131 358/http://www.simplynatural.org.uk/acatal og/Phyto_Soya_Night_Day.html [retrieved on 2011-09-22]
- "Estropiu' Bustine", Saninforma , 1 September 2010 (2010-09-01), XP002679815, Retrieved from the Internet: URL:http://www.saninforma.it/Sezione.jsp?i dSezione=13458&sez=prodotti&cat=dietetici- e-integratori&titolo=estropiu-bustine [retrieved on 2012-07-12]
- T. ASO: "Equol Improves Menopausal Symptoms in Japanese Women", THE JOURNAL OF NUTRITION, vol. 140, no. 7, 19 May 2010 (2010-05-19), pages 1386S-1389S, XP055007310, ISSN: 0022-3166, DOI: 10.3945/jn.109.118307
- NARUMOL PHOSRITHONG ET AL: "Molecular docking study on anticancer activity of plant-derived natural products", MEDICINAL CHEMISTRY RESEARCH, vol. 19, no. 8, 28 July 2009 (2009-07-28), pages 817-835, XP055007493, ISSN: 1054-2523, DOI: 10.1007/s00044-009-9233-5
- PEARSON K J ET AL: "Resveratrol Delays Age-Related Deterioration and Mimics Transcriptional Aspects of Dietary Restriction without Extending Life Span", CELL METABOLISM 20080806 US LNKD- DOI:10.1016/J.CMET.2008.06.011, vol. 8, no. 2, 6 August 2008 (2008-08-06), pages 157-168, XP002659775, ISSN: 1550-4131
- FAURE EVELYNE DRAPIER ET AL: "Effects of a standardized soy extract on hot flushes: a multicenter, double-blind, randomized, placebo-controlled study.", MENOPAUSE (NEW YORK, N.Y.) 2002 SEP-OCT LNKD- PUBMED:12218721, vol. 9, no. 5, September 2002 (2002-09), pages 329-334, XP002659776, ISSN: 1072-3714
- HEINEMANN LOTHAR AJ ET AL: "International versions of the Menopause Rating Scale (MRS)", HEALTH AND QUALITY OF LIFE OUTCOMES, BIOMED CENTRAL, LONDON, GB, vol. 1, no. 1, 30 July 2003 (2003-07-30), page 28, XP021008835, ISSN: 1477-7525, DOI: 10.1186/1477-7525-1-28 cited in the application
- ANONYMOUS: 'Einfluss von Estradiol, Genistein, Equol und Resveratrol auf verschiedene östrogensensitive Organe und Parameter der ovariektomierten Maus - eDiss', [Online] 03 February 2011, GEORG-AUGUST-UNIVERSITÄT ZU GÖTTINGEN, XP055130759 Retrieved from the Internet: <URL:https://ediss.uni-goettingen.de/handle /11858/00-1735-0000-0006-B17D-A?locale-attr ibute=en> [retrieved on 2014-07-22]
- ANONYMOUS: 'Latest On Alzheimer's Disease, Omega-3, Vitamin D, Soy - Life Extension What's Hot', [Online] 24 October 2009, XP055130745 Retrieved from the Internet: <URL:http://www.lef.org/whatshot/2009_10.ht m> [retrieved on 2014-07-22]
- S. JOY: 'The Isoflavone Equol Mediates Rapid Vascular Relaxation: Ca2+-INDEPENDENT ACTIVATION OF ENDOTHELIAL NITRIC-OXIDE SYNTHASE/Hsp90 INVOLVING ERK1/2 AND Akt PHOSPHORYLATION IN HUMAN ENDOTHELIAL CELL' JOURNAL OF BIOLOGICAL CHEMISTRY vol. 281, no. 37, 20 July 2006, pages 27335 - 27345, XP055130822 DOI: 10.1074/jbc.M602803200 ISSN: 0021-9258
- KLINGE CAROLYN M ET AL: "Resveratrol stimulates nitric oxide production by increasing estrogen receptor alpha-Src-caveolin-1 interaction and phosphorylation in human umbilical vein endothelial cells", FASEB JOURNAL, FED. OF AMERICAN SOC. FOR EXPERIMENTAL BIOLOGY, US, vol. 22, no. 7, 1 July 2008 (2008-07-01), pages 2185-2197, XP009134551, ISSN: 0892-6638, DOI: 10.1096/FJ.07-103366 [retrieved on 2008-02-22]
- MARIE LAGOUGE ET AL: "Resveratrol improves mitochondrial function and protects against metabolic disease by activating SIRT1 and PGC-1alpha", CELL, CELL PRESS, US, vol. 127, no. 6, 15 December 2006 (2006-12-15), pages 1109-1122, XP002688765, ISSN: 0092-8674, DOI: 10.1016/J.CELL.2006.11.013

## Description

### SCOPE OF THE INVENTION

This invention concerns a composition for the treatment and prevention of disorders related to menopause and/or aging of the human organism.

In particular, one aspect of this invention concerns a composition for the treatment of the problems/disorders associated with menopause, for example hot flushes, dismal mood/depression, insomnia, difficulty concentrating, general aging of the organism, aging of the skin, lowering of the metabolism, weight gain (excess weight).

Due to its capacity to counteract the general aging of the organism, the invention composition may also be used as an adjuvant in the treatment of aging of the skin, in the lowering of metabolism, and in the treatment of weight gain.

### STATE OF THE ART

Currently the products present on the market for the treatment of menopause disorders/problems are predominantly composed of soy isoflavones, red clover, and flax seed. The most widely known raw material to be used for menopause is soy. For soy, however, a legislative limit on the daily quantities that products may contain does exist in Italy. Specifically, the current legislation requires that 80 mg/day of soy isoflavones not be exceeded and the prevalent medical class believes that soy does not function efficaciously in the treatment of said disorders within this limit due to a problem of quantity, even if there is second portion of the medical class which believes that even these quantities may be effective.

From the perspective of better understanding the conflicting data in the literature relative to soy, in recent years the scientific community identified a new molecule, equol, which derives from the metabolism, in the intestine, of daidzein, a soy isoflavone. It was thus learned that the population may be subdivided into equol-producers or non equol-producers depending on whether the right bacteria are present in the intestine to carry out the above-said transformation. Equol's importance would seem to reside in the fact that it is similar to estradiol (the female hormone estrogen), it therefore mimics estradiol's activity, but at the same time it does not produce the side effects and contra-indications of estrogens.

Studies were thus conducted on fermented soy, which consequentially is naturally enriched with equol; these studies have demonstrated the effectiveness of the substance in reducing the symptoms associated with menopause at low doses (Takeshi et al, "Equol improves menopausal symptoms in Japanese women", The Journal of Nutrition; Supplement: equol, Soy and Menopause, 1386-1389).

WO 01/70212 discloses a composition comprising trans-resveratrol and other components such as soy isoflavones for treating or reducing the symptoms of menopause.

In the article "Effect of a novel dietary supplement on skin aging in post-menopausal women" in the name of Lange Skovgaard G.R. et al. in European Journal of clinical nutrition XX, vol. 60, 1 Jan. 2006, pages 1201-1206, it is disclosed a composition containing a soy extract, fish protein polysaccharides, extracts from white tea, grape seeds tomato, vitamins C and E and chamomile extracts as well as the use of said composition as a dietary supplement for reducing skin aging and post-menopausal women.

The publication available on internet Simply Natural "Phyto Soya Night & Day", Simply Natural Health Supplement & Homebrew Specialists, 28 August 2008 discloses a nutritional supplement for treating women in menopause containing a standardised soya extract and a grape skin and seed extract.

It is also known a food supplement called "Estropiù" (Saninforma, 1 Sept. 2010) which is a mixture of phytoestrogens extracted from soya, Polygonum, Passiflora and including mineral salts and which is useful for treating pre-menopausal symptoms in women.

The need to develop alternative and better products for treating menopause problems/disorders is still felt however.

### SUMMARY OF THE INVENTION

One of the purposes of the invention consists in preventing and/or in mitigating the effects on the human organism brought on by menopause.

In accordance with a first aspect this invention provides a composition for use as defined in appended claim 1.

The Applicant discovered the association of equol and resveratrol causes a synergic antioxidizing action at the level of certain cellular processes at the base of the disorders and/or of the symptoms associated with menopause and with cellular aging.

In particular, the inventors of this invention discovered that resveratrol, for example originating from an extract of *Vitis vinifera,* associated with equol, produces the synergic effect of activating the SIRT genes that code for sirtuin, enzymes capable of improving the cell efficiency counteracting the processes of oxidative stress that typically occur in menopause and/or in the aging processes of the human organism.

In certain embodiments, the invention composition is a pharmaceutical composition.

In some embodiments, the invention composition is a nutraceutical composition.

In certain embodiments, the invention composition is a dietetic product or a food supplement.

In some embodiments, the invention composition is a food product meant for animals.

In one embodiment, the resveratrol of the composition is obtained from *Vitis vinifera.*

In certain embodiments, the daidzein metabolite is equol obtained through fermentation in the presence of non-pathogenic microorganisms, in particular bacteria, that belong to an equol-producing strain.

In certain embodiments, the equol of the composition is obtained through a fermentative process comprising the transformation of daidzein into the inactive metabolite hydroxydaidzein, and the transformation of this metabolite into O-desmethylangolensin and equol.

In some embodiments, the invention composition comprises an extract of fermented soy with a total isoflavone content from 20 to 60%, in which the quantity of equol ranges from 5 to 20% and a *Vitis vinifera* extract with a resveratrol content from 5 to 15.2%.

In this invention, when indicating the resveratrol content of the extract of *Vitis vinifera,* commonly known as "grapes," it is understood to be the sum of monomers and oligomers, in which the resveratrol monomers amount to no less than 3 mg.

In accordance with a second aspect, this invention concerns the non-therapeutic use of a composition as defined in appended claim 11.

Under a third aspect, the invention concerns the non-therapeutic use of a composition as defined in appended claim 12.

### BRIEF DESCRIPTION OF THE FIGURES

This invention shall be described in detail further on with reference to the figures, in which:
Figure 1 shows the results of the Mitotracker staining in the HUVEC endothelial cells of example 2;
Figure 2 shows the results of the SIRT1 protein expression and SIRT1 enzymatic activity in the HUVEC cells of example 2;
Figure 3 shows the results of the protein expression of porin and of the components of the respiratory chain in the HUVEC cells of example 2;
Figure 4 shows the results relative to the genetic expression of the mitochondrial biogenesis factors PGC-1α, Nrf-1, and Tfam in the HUVEC cells of example 2;
Figure 5 shows the results relative to adipogenesis in the preadipocytes of example 2;
Figure 6 refers to the results of the Mitotracker staining and the quantification of the mitochondrial DNA;
Figure 7 refers to the measurement of the mRNA expression of mitochondrial biogenesis factors via QRT-PCR;
Figure 8 refers to the quantification of the lipid content.

### DETAILED DESCRIPTION OF THE INVENTION

In accordance with a first aspect, the invention is directed to a composition for the use as defined in claim 1.

The inventors of this invention have observed that resveratrol acts synergically with equol inducing a phytoestrogenic activity in the human or animal organism. This activity is particularly perceptible in female subjects in menopause.

The term "menopause" is understood to be the time of the cessation of spontaneous menstruation that typically coincides with the end of the physiological reproductive life. This phenomenon, in particular in western women, may occur at an age between 48 and 53 years, inclusive.

Menopause is typically very evident after an amenorrhea, a period of absence of menstruation of at least 6 months, in a female subject who has reached the average age of onset.

From a biological perspective, menopause consists in the cessation of the activity of the ovary, the female gonad, located at the side of the uterus, which produces the female germ cells or oocytes and that secrete estrogens, the female hormones.

With menopause, estrogen levels typically reach very low values while there is a progressive increase of FSH.

Within the scope of this invention, the term menopause syndrome is understood to be the disorders and the symptoms that accompany menopause and that typically originate from the variation of female hormone levels, and estrogens in particular, which occur during or in the period prior to menopause.

Equol ((3S)-3-(4-Hydroxyphenyl)-7-chromanol or 4',7-lsoflavandiol) present in the invention composition is an active metabolite obtained from daidzein (7-Hydroxy-3-(4-hydroxyphenyl) chromen-4-one or 4',7-Dihydroxyisoflavone), an isoflavonoid in aglyconic form naturally present in soy and in similar non-fermented products.

Typically, the equol used in the invention composition is not present as such in the isoflavonoids, in the soy or soy shoots found in nature.

In certain embodiments, the equol of the invention composition is obtained through metabolism or fermentation of daidzein by equol-producing microorganisms that are typically non-pathogenic bacteria, such as for example bifidus bacteria, lactobacilli, which may be present in the intestinal ecosystem of certain individuals. In some embodiments of the invention composition, equol is obtained through the fermentation of daidzein with a non-pathogenic, equol-producing microorganism.

In certain embodiments, the equol-producing microorganisms are *Pediococcus acidilactici* bacteria, preferably of the genus *Lactococcus lactis,* particularly as described in the Italian patent application MI2011A001829 filed on October 7, 2011 on behalf of the Applicant himself.

In certain embodiments, the equol of the invention is obtained from the fermentation of microorganisms belonging to the genus *Enterococci thailandicus,* as described in the Italian patent application MI2011A001283 dated July 8, 2011 on behalf of the Applicant.

In certain embodiments, the equol of the invention composition is contained in a fermented-soy extract.

Typically, equol exists in two mirror-image isoforms, S-equol and R-equol.

The form produced by certain equol-producing bacteria, present in particular in the intestinal microflora of certain individuals, is S-equol. This represents the most active form of equol in terms of biological activity.

Typically, R-equol is the form obtained through chemical/fermentative synthesis starting from daidzein.

In certain embodiments, the equol present in the invention composition is the S isomer.

Equol has a bonding affinity in relation to alpha and beta estrogenic human receptors much greater than daidzein (from ten to eighty times) and induces the transcription much more strongly than any other isoflavonoid (N. Sathyamoorthy et al., Eur. J. Cancer, 1977, 33, 2384-2389; G. Kuiper et al., Endocrinology, 1998, 139, 4252-4263; K. Morito et al., Biol. Phatm. Bull., 2001, 24, 351-356; D. Kostenac et al., J. Agr. Food Chem., 2003, 51, 7632-7635; R.S. Muthyala et al., Bioorg. Med. Chem., 2004, 12, 1559-1567).

Equol, moreover, again with respect to the other isoflavonoids, has a significantly stronger antioxidant power (J.H. Mitchell et al., Arch. Biochem. Biophys., 1998, 360, 142-148).

The soy extracts with measured isoflavone content present on the market do not exhibit detectable levels of equol with standard methods.

Preparations based on fermented soy are available on the market, both as foods (natto, tempeh) and as food supplements, in which the bacteriological fermentation action is capable of increasing aglyconic isoflavone levels. In this preparations also, no significant quantities of equol are observed.

Typically, the fermentation of the soy via specific equol-producing strains of bacteria or strains conditioned to produce equol is capable of producing significant quantities of equol starting from daidzein.

Daidzein is one of the major isoflavonoids. Daidzein is present in soy mainly in the biologically inactive forms of beta-glucoside, acetyl-glycoside, or malonyl-glycoside. It cannot be absorbed by the human intestine in these forms which, after ingestion, are hydrolyzed with aglycone only in small quantities at the level of the small intestine and, then, absorbed by the intestinal epithelium itself (A.J. Day et al., FEBS Lett., 2000, 468, 166-170). A large non-hydrolyzed fraction reaches the colon where the forms <glycosylated, sulfated, and glucuronidated are deconjugated by the enzymes produced by the microorganisms (A.J. Day et al., 2000, ref. cit.; M. Richelle et al., J. Nutr., 2002, 132, 2587-2592; J. Chen et al., J. Pharmacol., Exp. Ther., 2003, 304, 1228-1235; K.D.R. Setchell et al., Am. J. Clin. Nutr., 2003, 77, 411-419).

In particular, the glycosidases of the bacteriological flora of the intestine cleave the beta-glycosidic bond, liberating the aglycone. The aglycones are then absorbed and subject to enterohepatic circulation or to further metabolism by the intestinal bacteria (H. Wiseman, Proc. Nutr. Soc., 1999, 58, 139-146).

Upon returning to the intestine, they are then transformed in the colon by the microorganisms present:
- genistein is converted into dihydroxygenistein
- daidzein is converted into dihydroxydaidzein (DHD), Equol and O-desmethylangolensin (O-DMA)

The capacity to carry out these transformations varies depending on single individuals. While approximately 80-90% of the population is able to produce O-desmethylangolensin, only 30-50% is able to synthesize equol from daidzein.

In certain embodiments, the soy-fermentation process results in an isoflavone content equal to 20-60%, mainly represented by genistein and daidzein. In certain embodiments, the invention composition contains equol in the form of a fermented-soy extract.

In certain embodiments, the fermented-soy extract, with a total isoflavone content in the interval from 20 to 60%, of which equol is 5 to 20% of the invention composition, corresponds to an interval from 1 mg to 100 mg of equol in the final composition.

In some embodiments, the soy extract of the invention contains from 1 to 30 mg of equol, normally about 9.6 mg of equol.

In certain embodiments, the invention composition includes a quantity of fermented-soy extract from 50 to 1000 mg, preferably from 100 to 400 mg, more preferably about 200 mg of fermented-soy extract.

The invention composition also contains resveratrol, preferably in the form of a *Vitis vinifera* extract. In certain embodiments, the *Vitis vinifera* extract has a resveratrol content from 5 to 15.2%.

Resveratrol, or trans-3,5,4'-trihydroxystilbene, is a polyphenolic compound present in various quantities in diverse species of plants, including grapes, peanuts, and Polygonum cuspidatum.

In this invention, the resveratrol preferably derives from an extract of grapes, typically from *Vitis vinifera.* The resveratrol content includes both monomers and oligomers thereof.

In some embodiments of the invention composition, the *Vitis vinifera* extract with a resveratrol content in the interval from 5 to 15.2% corresponds to an interval from 1 mg to 1000 mg of resveratrol in the final composition.

In certain embodiments, the *Vitis vinifera* extract of the invention contains from 1.25 to 5 mg of resveratrol, for example approximately 3.4 mg of resveratrol monomer or 7.5 mg of resveratrol as the sum of monomers and oligomers.

In some embodiments, the invention composition includes a quantity of *Vitis vinifera* extract from 10 to 200 mg, preferably from 20 to 100 mg, for example, approximately 25 mg of *Vitis vinifera* extract.

Certain embodiments of the invention composition contain a fermented-soy extract with a total isoflavone content in the interval from 20 to 60%, of which equol ranged from 5 to 20% and a *Vitis vinifera* extract with a resveratrol content in the interval from 5 to 15.2%.

The invention composition may be administered orally and in the form of solid and liquid preparations. When the preparation is solid, the composition may be administered in the form of tablets, capsules, powders, or pastilles, preferably in tablet form. When the preparation is liquid, the invention composition may be administered in the form of a solution, suspension, drops, or syrup.

In some embodiments, the invention composition contains a fermented-soy extract with a total isoflavone content in the interval from 20 to 60%, of which equol is from 5 to 20% and a *Vitis vinifera* extract a resveratrol content in the interval from 5 to 15.2%.

In certain embodiments, the invention composition includes one or more edible carriers and/or excipients.

For the purpose of this invention, the term edible is understood to signify food-grade materials that are approved by the Regulatory Authorities for use in pharmaceutical, food, nutritional, and/or dietetic applications.

In certain embodiments, the invention composition also includes excipients chosen from the group consisting in bulking agents, anti-caking agents, stabilizing agents, thickening agents, humectants, tableting agents, and colorants commonly used within the scope of formulation techniques for pharmaceutical compositions or nutritional or dietetic preparations.

In the embodiments in which the invention composition is in tablet form, it may be coated with glazing agents.

In certain embodiments, the invention composition also includes plant extracts, for example of Pueraria lobata, and also, or alternatively, vitamins and/or minerals.

Under another aspect, the invention concerns a composition in accordance with any of the embodiments previously described for use as a medicinal, in particular for the use in the treatment of menopause symptoms/disorders, associated especially with hormonal changes consequential to the onset of menopause.

In certain embodiments, the menopause disorders include hot flushes that typically manifest themselves as a sudden sensation of warmth on the dermis of the face, neck, and thorax, that may be accompanied by redness and sudation of the skin.

Typically, these hot flushes may have an average duration of approximately 3 minutes and a frequency that generally decreases progressively over the course of years until it nearly disappears after approximately 10 years after the last menstruation.

In certain embodiments, the invention composition is used in the treatment or prevention of genitourinary tissue alterations associated with a drop in circulating levels of estrogens.

In certain embodiments, the invention composition is applied in the treatment or in the prevention of the following symptoms or disorders: dryness of the vagina, associated with burning or inconvenience during sexual intercourse, cystitis, burning sensation during urination, pelvic pains, frequent urination, and forms of urinary incontinence.

For example, the composition is applicable in cases where the dermis tends to thin out, and/or there is a tendency for relative dryness and there may be a slight increase of vellus hair in certain zones of the face.

In certain embodiments, the composition is applicable in the cardiovascular disorders related to menopause in which the incidence of ischemic phenomena increase, and the lipid profile tends to worsen (increase of cholesterol and triglycerides, reduction of HDL).

In certain embodiments, the composition can be applied in cases of menopause in which the bones tend to progressively decrease their density or in case of osteoporosis or in cases of increased risk of fracture, especially in relation to the vertebrae and femur.

The composition may also be applied in menopause associated with worsening of depressive moods.

In certain embodiments, the invention composition is applied in the treatment or in the prevention of the symptoms or disorders in the premenopausal phase.

The term premenopausal period is understood to be the period that precedes menopause. Typically, there is a progressive irregularity of the menstrual cycle in this period followed by the almost complete cessation of menstrual flow.

In certain embodiments, the invention composition is applied in the treatment or in the prevention of the disorders of early menopause.

The term early menopause is understood to be a menopause that manifests itself spontaneously in female subjects under the age of 40.

Again, under an additional aspect, the invention concerns the invention composition for the use in the treatment of general aging of the organism.

Under another aspect, the invention comprises a nutraceutical formulation containing the invention composition, preferably for use in the treatment of menopause problems/disorders and/or in the treatment of general aging of the organism, associated in particular with hormonal changes consequential to the occurrence of menopause.

The invention composition is in fact capable of specifically activating mitochondrial biogenesis in different tissues. The invention composition is also capable of activating specific cellular-transcription factors that induce anti-inflammatory and antioxidant proteins. By means of these actions, the invention composition is effective in promoting the viability and the functions of the cells, tissues, and of the organism, and is particularly effective in protecting the endothelial cells, skin cells, and nervous system cells against deterioration, against death due to degenerative processes and in aging in general. The invention composition is also active in terms of antiobesity effects, inducing the inhibition of adipogenesis and proapoptotic effects in human adipocytes.

All of these results of effectiveness will be evident from the experimental part that follows which presents experimental results in terms of an increase in mitochondrial biogenesis, an increase in lipolysis, an induction of apoptosis in mature adipocytes, and the activation of SIRT 1 in endothelial cells.

These experimental results may be attributed to benefits such as:
- general anti-aging activity on the organism
- cutaneous anti-aging action
- inhibition of the lowering of metabolism with the resulting inhibition of weight gain
- modulation of estrogen receptors with the reduction of typical menopause symptoms.

Below are examples of invention embodiments and the evaluation of the invention's effects, provided for the sake of example and not restrictively for the invention itself.

### EXAMPLES

### Example 1:

The ingredients indicated in the table below were used in the quantities shown to prepare the invention composition.

**Table 1: Composition of a tablet of the invention.**

| **Description** | **Active Ingredient of the raw material** | **mg/un.** | **g/100 g** |
|---|---|---|---|
| Bulking agent: microcrystalline cellulose | | 207.000 | 31.364 |
| Fermented-soy extract (equol content: 4-5.6%) | 40% isoflavones and total derivatives | 200.000 | 30.303 |
| Bulking agent: dicalcium phosphate | | 150.000 | 22.727 |
| *Vitis vinifera* extract Resveratrol content: 5-15.2% | 30% monomers and oligomers | 25.000 | 3.788 |
| Anti-caking agent: crosslinked sodium carboxy methyl cellulose | | 16.000 | 2.424 |
| Glazing agent: hydroxypropyl methyl cellulose | | 13.000 | 1.970 |
| Anti-caking agent: Glyceryl behenate | | 12.000 | 1.818 |
| Stabilizing agent: polyvinylpyrrolidone | | 10.000 | 1.515 |
| Anti-caking agent: magnesium stearate of vegetable origin | | 10.000 | 1.515 |
| Anti-caking agent: silicon dioxide | | 10.000 | 1.515 |
| Colorant: titanium dioxide | | 3.000 | 0.455 |
| Glazing agent: microcrystalline cellulose | | 2.000 | 0.303 |
| Glazing agent: stearic acid | | 2.000 | 0.303 |
| | | | |
| **Total** | | 660.00 | 100.000 |

The ingredients above were mixed and sifted on a 1.0 mm mesh. All components with the exception of magnesium stearate were been transferred in the mixer and mixed for 10 minutes. Magnesium stearate was then transferred in the mixer and mixed for 5 minutes.

The material was compressed to a theoretical weight of 640 mg/tablet (17.5 x 8.8 mm shape without snap tab). The tablets were ten coated with a dispersion of the glazing agents indicated in table 1.

660 mg tablets having the following characteristic were obtained:

| Tablet weight | **660 mg** | Validity | **36 months** |
|---|---|---|---|
| Appearance | **oval** | | |
| Color | **white** | | |

### EXAMPLE 2

Evaluating the effects of the invention composition.

The effects of the invention composition were evaluated using the composition of example 1.

The effects were compared with the effects from equol alone and from resveratrol alone in the same quantities as the invention composition.

Mitochondria are flexible and complex cellular entities that play crucial roles in pathological and normal cellular conditions. Mitochondrial biogenesis is an elaborate cellular process that relies on the strong bond of diverse regulatory controls, from gene transcription to the site-specific production of proteins. The pathways that govern mitochondrial biogenesis have recently emerged as potential therapeutic targets to improve endothelial dysfunction and vascular disorders observed in metabolic diseases (Ren. J., Pulakat L, Whaley-Connell A, Sowers JR. Mitochondrial biogenesis in the metabolic syndrome and cardiovascular disease. J Mol Med. 2010 Oct; 88(10):993-1001).

Mitochondrial mass was measured in HUVEC endothelial cells using the Mitotracker red staining technique as follows.

The HUVEC cells were subdivided into three groups, which were treated with resveratrol, 10 µmol/l for 48 h, equol 10 µmol/l for 48 h, and with the composition containing resveratrol and equol, 10 µmol/l for 48 h, respectively. The mitochondrial mass in HUVEC was determined selectively loading mitochondria with Mitotracker fluorescent red dye (Invitrogen, Carlsbad, CA). Fluorescent calcein (green) and Hoechst 33258 (blue) dyes were used to stain the cytoplasm and the nuclei, respectively. Optical sections of HUVEC were captured at x60-magnification and the mitochondrial density-area ratio was calculated with respect to cytoplasmatic volume using the Zeiss Axiovision imaging software. Only cells with intact cytoplasmatic calcein stain were included in the analysis.

The mitochondrial DNA content in the HUVEC cells was then measured as follows.

The total DNA was isolated from the HUVEC cells (DirectPCR; Viagen Biotech). The number of mitochondrial DNA copies (mtDNA) was determined via QRT-PCR as described in Addabbo F, Ratliff B, Park HC, Kuo M, Ungvari Z, Csiszar A, Krasnikov B, Sodhi K, Zhang F, Nasjletti A, Goligorsky MS. The Krebs cycle and mitochondrial mass are early victims of endothelial dysfunction: proteomic approach. Am J Pathol 174: 34-43, 2009.

Using cytochrome oxidase III and beta-actin as markers for the number of mtDNA and genomic DNA copies, respectively. The impact of the treatment with resveratrol, equol, and the invention composition containing both resveratrol and equol was evaluated.

### SIRT1 activity assay

SIRT1 nuclear activity was measured in cells treated with resveratrol, equol, and the invention composition as described previously (Ferrara N, Rinaldi B, Corbi G, Conti V, Stiuso P, Boccuti S, Rengo G, Rossi F, Filippelli A. Exercise training promotes SIRT1 activity in aged rats. Rejuvenation Res. 2008 Feb; 11(1):139-50).

### Measurement of the protein expression of constituents of the electron transport chain.

To explain the effect of resveratrol, equol, and of the invention composition (resveratrol + equol) on the protein expression of constituents of the electron transport chain Western blotting was performed as described in Nicoletti VG, Caruso A, Tendi EA, Privitera A, Console A, Calabrese V, Spadaro F, Ravagna A, Copani A, Stella AM. Effect of nitric oxide synthase induction on the expression of mitochondrial respiratory chain enzyme subunits in mixed cortical and astroglial cell cultures. Biochimie. 1998 Oct; 80(10):871-81. Direct primary antibodies against complex II, complex V (Molecular Probes/Invitrogen) and cytochrome oxidase were used (COX-IV, no. 4844; Cell Signaling). The levels of porin, the most abundant protein of the outer mitochondrial membrane, were also measured. Because of its abundance, porin is often used as a marker for the cell mitochondrial mass. Anti--β-actin (no. 6276; Abcam) was used for normalization purposes.

### Measuring the mRNA expression of mitochondrial biogenesis factors via QRT-PCR.

The QRT-PCR technique was used to determine the effect of resveratrol, equol, and of the invention composition (resveratrol + equol) (10 mol/l, for 24 hours) on the mRNA expression of mitochondrial biogenesis factors: nuclear respiratory factor (Nrf-1), mitochondrial transcription factor A (Tfam), and Peroxisome proliferator-activated receptor (PPAR)-α coactivator-1 (PGC-1 α) in HUVEC using Light Cycler (Roche Molecular Biochemicals) as described in Scapagnini G, Butterfield DA, Colombrita C, Sultana R, Pascale A, Calabrese V. (2004) Ethyl ferulate, a lipophilic polyphenol, induces HO-1 and protects rat neurons against oxidative stress. Antioxid Redox Signal. 6(5):811-8.)

The total RNA was isolated with the isolation kit with Mini RNA (Zymo Research, Orange, CA) and was inverse-transcribed using Superscript III RT (Invitrogen) as described in Scapagnini G, D'Agata V, Calabrese V, Pascale A, Colombrita C, Alkon DL, Cavallaro S. (2002) Gene expression profiles of heme oxygenase isoforms in the rat brain Brain Res. 954(1): 31-39.

The effectiveness of the PCR reaction was determined employing a series of standard sample dilutions. The housekeeping gene HPRT was used for internal normalization. The PCR reaction reproducibility was determined via melting point analysis and viewing the product on 2% agarose gel.

### Adipocyte viability and apoptosis assays.

Tests were performed in 96-well plates. For the mature adipocytes, the cells were seeded (5000 cells/well) and grew until maturing as described further above. The cells were seeded (2500 cells/well) and the assay was performed 4 days after seeding for the preadipocytes. Preadipocytes or mature adipocytes were incubated with 0.2% dimethyl sulfoxide (DMSO) or test compounds for 24 and 48 hours. The cellular vitality assay was conducted in accordance with the instruction manual. Absorbance was measured at 490 nm in a plate reader (mQuant, Bio-Tek Instruments) to determine the formazan concentration, which is proportional to the number of live cells. The apoptosis detection kit ApoStrand ELISA was used to measure the apoptosis content. The cells were grown in 96-well plates, treated with the test compounds for 24 and 48 hours, and tested in accordance with the instruction manual. The assay selectively detects single-stranded DNA, which appears in apoptotic cells, but not in necrotic cells or cells with DNA cleavage in the absence of apoptosis. Assays were performed at least twice for 6 replicas for each treatment.

### Quantification of the lipid content.

The lipid content was quantified using the commercially available assay reagent AdipoRed. In short, post-confluent preadipocytes were grown in 96-well plates and incubators with 0.2% DMSO or test compounds during the adipogenic phase, and on the sixth day they were tested for lipid content in accordance with the instruction manual. The experiments were performed with at least 6 replicas per treatment and repeated 3 times.

### Lipolysis assay.

To determine the lipolysis content induced by the test compounds, the mature adipocytes were treated with 0.2% DMSO or the test compounds for 5 h, and the free glycerol released was tested using an adipocyte-lipolysis assay kit (Zen-Bio) and in accordance with the instruction manual. The experiment was repeated twice with at least 4 replicas.

### Results

The invention composition induces mitochondrial biogenesis in endothelial cells.

This action is associated with the treatment of age-associated diseases and with correlated symptoms such as menopause (Tina Wenz Mitochondria and PGC-1α in aging and age-associated diseases, in the Journal of Aging Research Vo. 2011 Art. ID810619, pages 1-12).

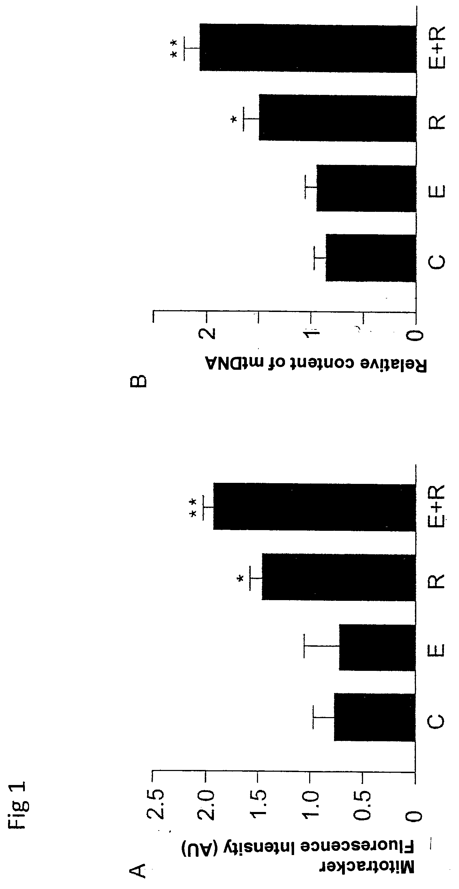

The Mitotracker staining showed that mitochondria were located in the perinuclear region in HUVEC. The density-area ratio with respect to cytoplasmatic volume in Mitotracker-labeled endothelial cells increased significantly after treatment with resveratrol (Fig. 1). The analysis of the Mitotracker intensity in HUVEC confirmed that treatment with resveratrol significantly increased the mitochondrial mass in treated cells compared with non-treated cells (Fig. 1). Equol alone was not effective in terms of activating the mitochondrial mass, however the invention composition (resveratrol + equol) was significantly more effective compared with resveratrol alone (Fig. 1).

### The invention composition also induces SIRT1 in endothelial cells.

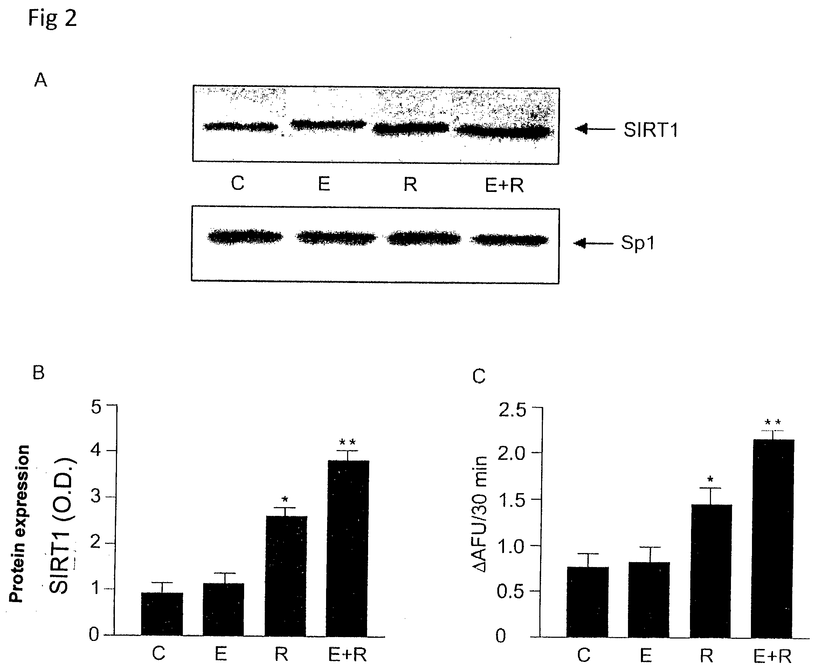

The invention composition proved capable of activating the protein expression of SIRT1 and of increasing the SIRT1 enzymatic activity in HUVEC endothelial cells (Fig 2). Equol was not effective in terms of activating SIRT1, however the invention composition was significantly more effective in comparison with resveratrol alone (Fig. 2).

The composition activated the constituents of the mitochondrial electron transport chain in endothelial cells.

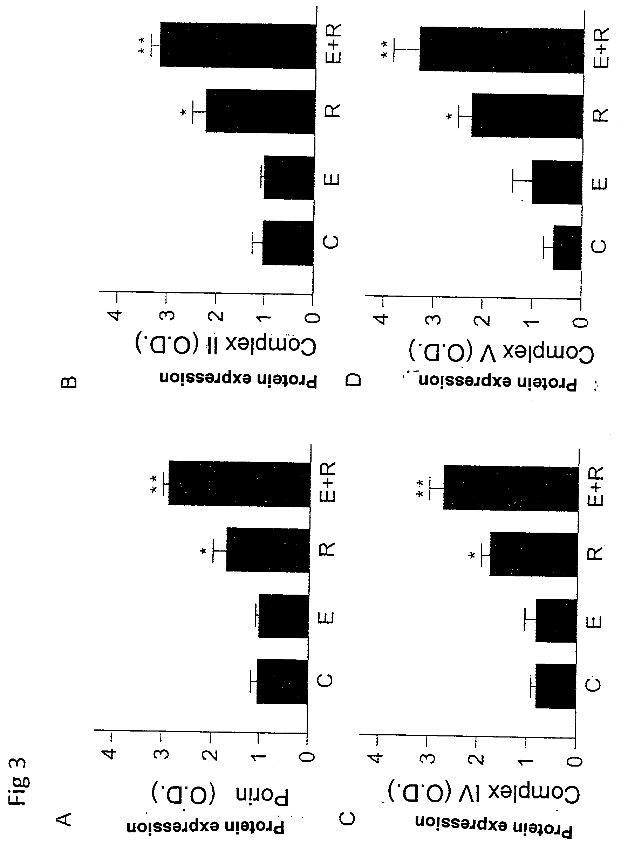

Through Western blotting it was shown that in the cells treated with the invention composition, porin following the instruction manual had increased significantly when compared with untreated HUVEC (Fig. 3A). The porin levels (which control the diffusion of small metabolites across the outer membrane) were correlated with the cellular mitochondrial volume. The expression of complex II, complex IV, and complex V in HUVEC cells (Fig. 3B, C, D) also increased through treatment with the invention composition.

The invention composition activated the mitochondrial biogenesis factors.

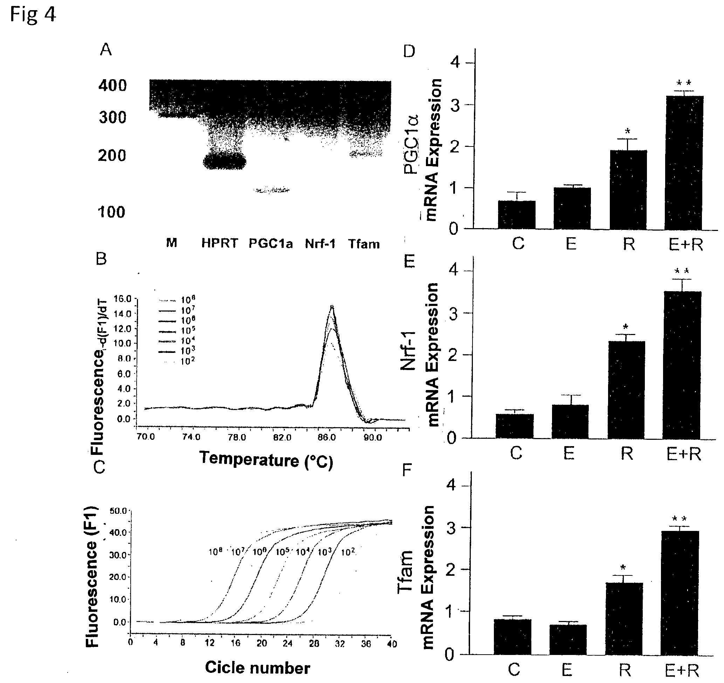

Mitochondrial biogenesis involves the integration of multiple transcriptional pathways, which control both nuclear and mitochondrial gene expression. The PPAR-gamma coactivator (PGC-1α), and the transcription factors Nrf-1 and Tfam are considered key regulators of mitochondrial biogenesis in multiples tissues. Measurements via QRT-PCR revealed that the expression of the mitochondrial biogenesis factors PGC-1α, Nrf-1, and Tfam in HUVEC cells (Fig. 4) had increased significantly via treatment with the invention composition.

### Cellular viability of preadipocytes and mature adipocytes.

Equol was unable to decrease cellular viability in preadipocytes at 24 h and at 48 h. Resveratrol slightly decreased cellular viability (~ 20% at 24 h and ∼ 28% at 24 h). The invention composition further decreased cellular viability by 42% at 24 h and by 57% (P = 0.001), after a 48 hours of treatment in accordance with Table 2 below.

**Table 2**

| | **Preadipocyte** | | **Mature Adipocyte** | |
|---|---|---|---|---|
| **Treatment** | **24 h** | **48 h** | **24 h** | **48 h** |
| **C** | **0.0 ± 2.2** | **0.0 ± 3.7** | **0.0 ± 1.8** | **0.0 ± 2.3** |
| **E** | **5.5 ± 3.4** | **3.2 ± 1.6** | **5.8 ± 3.4** | **1.2 ± 0.8** |
| **R** | **-1 9.2 ± 3.2** | **-28.5 ± 2.4** | **-16.6 ± 4.1** | **-21 ± 2.3** |
| **E + R** | **-42.6 ± 1.5** | **-57.4 ± 3.2** | **-46.4 ± 2.6** | **-58.8 ± 2.7** |

Similarly, the mature adipocytes were treated with the invention composition (resveratrol and equol), and resveratrol and equol as individual compounds for 24 and 48 hours. Equol was unable to decrease viability. Resveratrol alone slightly decreased cellular viability, while the invention composition appreciably decreased cellular viability by 46% (P = 0.001) after 24 hours by 58% (P = 0.001) after 48 hours (Table 2).

### Induction of apoptosis in mature adipocytes

Neither resveratrol nor equol increased apoptosis as individual treatments, whereas the association of the compounds increased apoptosis by 32% at 24 h and by 56% at 48 h (Table 3).

**Table 3**

| **Treatment** | **24 h** | **48 h** |
|---|---|---|
| | % change | |
| C | 0.0 ± 3.2 | 0.0 ± 3.8 |
| E | -5.6 ± 1.4 | -22 ± 2.6 |
| R | -7.2 ± 2.3 | -18 ± 2.7 |
| E+R | 32 ± 1.5 | 56 ± 3.4 |

### Inhibition of lipid accumulation

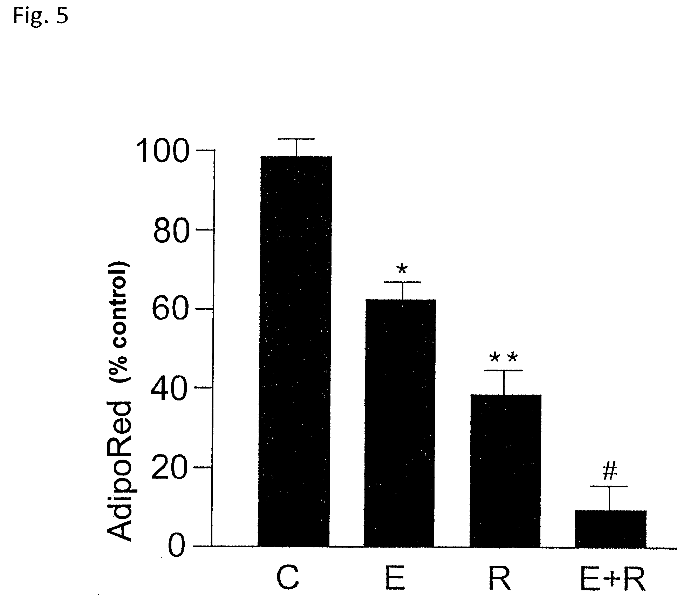

In maturing preadipocytes, preliminary experiments with resveratrol and equol showed that the combined effect on adipogenesis was very powerful. The effect on adipogenesis was much more powerful than the effect on viability, indicating a true decrease in lipid accumulation (fig 5).

### Effects on lipolysis

Neither resveratrol nor equol induced lipolysis as individual treatments whereas the combination increased it by 40% (P = 0.01).

Therefore, the invention composition induced mitochondrial biogenesis in human endothelial cells. The formation of new mitochondria was associated with the activation of SIRT1 and the induction of specific mitochondrial biogenesis factors. Moreover, the invention composition had been shown to be capable of exerting an increased effect on apoptosis induction and on adipogenesis inhibition in adipocytes. The invention composition had likewise showed that the decrease in the number and dimension of the mature adipocytes entailed the loss of lipids and the loss of cells via apoptosis.

The results reported above support the inventive nature of the composition that increased the mitochondrial content in endothelial cells (Fig. 1). The increased mitochondrial biogenesis in the cells treated with the invention composition was also indicated by the increased cellular mtDNA content (Fig. 1) and by the increased expression of proteins of the respiratory-chain components (Fig. 3). Multiple mechanisms could explain that the invention composition induced mitochondrial biogenesis and its contribution to cellular health. To determine if the increased number of mitochondria in endothelial cells treated with the invention composition was a consequence of the induction of mitochondrial biogenesis factors, QRT-PCR was used to examine the expression of Nrf-1, Tfam, and PGC-1α. Nrf-1 activates the transcription of many coded nuclear elements of the electron transport chain and also regulates Tfam, responsible for the transcription of mtDNA-coded genes. The regulatory function of Nrf-1 and of other mitochondrial biogenesis factors is modulated by PGC-1α. It was shown that resveratrol induced Nrf-1, Tfam, and PGC-1α in human endothelial HUVEC cells (Fig. 4). It was also established that the altered expression of these factors modulated the mitochondrial biogenesis activity. Furthermore, the invention composition induced SIRT1 (Fig. 2) and SIRT1 similarly regulated multiple pathways involved in mitochondrial biogenesis in endothelial cells, including the fact that SIRT1 could also directly deacetylate PGC-1α, increasing its activity.

### EXAMPLE 3

The HUVA cells were subdivided into 6 groups, which were treated with 10 µmol/l equol for 48 h, 10 µmol/l daidzein for 48 h, 10 µmol/l of resveratrol for 48 h, fermented soy with a (18%) daidzein content and resveratrol for total of 10 µmol/l of said active ingredients for 48 h, and with the invention composition containing fermented-soy extract with an equol content of 4-5.6%, and *Vitis vinifera* extract with a resveratrol content of 5-15.2% for a total of µmol/l of said active ingredients for 48 h, and finally an untreated control group, respectively.
A) The mitochondrial mass in HUVA was determined selectively loading mitochondria with Mitotracker fluorescent red dye (Invitrogen, Carlsbad, CA). Fluorescent calcein (green) and Hoechst 33258 (blue) dyes were used to stain the cytoplasm and the nuclei, respectively. Optical sections of HUVA were captured at ×60 magnification and the mitochondrial density-area ratio was calculated with respect to cytoplasmatic volume using the for Zeiss Axiovision imaging software. Only cells with intact cytoplasmatic calcein stain were included in the analysis.
B) The mitochondrial DNA content in the HUVA cells was then measured as follows. The total DNA was isolated from the HUVA cells (DirectPCR; Viagen Biotech). The number of mitochondrial DNA copies (mtDNA) was determined via QRT-PCR as described in Addabbo F, Ratliff B, Park HC, Kuo M, Ungvari Z, Csiszar A, Krasnikov B, Sodhi K, Zhang F, Nasjletti A, Goligorsky MS. The Krebs cycle and mitochondrial mass are early victims of endothelial dysfunction: proteomic approach. Am J Pathol 174: 34-43, 2009.) using cytochrome oxidase III and beta-actin as markers for the number of mtDNA and genomic DNA copies, respectively. The impact of the treatment with equol, daidzein, resveratrol, fermented soy with a measured content of daidzein and resveratrol, and the invention composition containing both resveratrol and equol was evaluated.

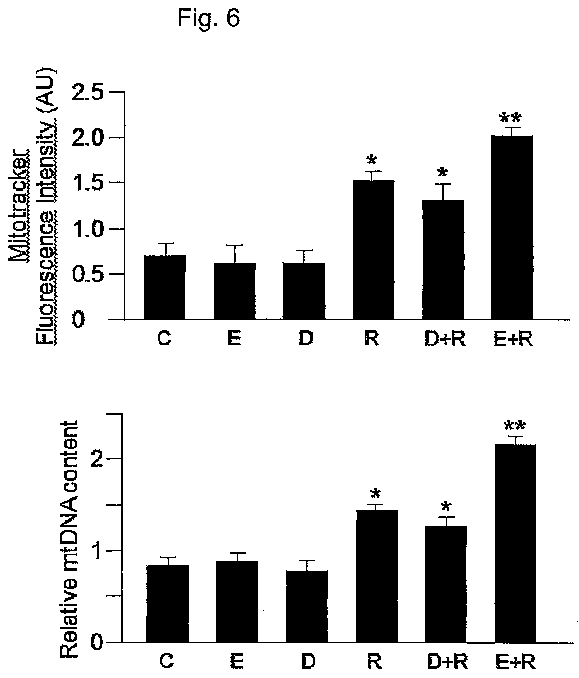

The results obtained are shown in figure 6.

### EXAMPLE 4

Measuring the mRNA expression of mitochondrial biogenesis factors via QRT-PCR.

The QRT-PCR technique was used to determine the effect of equol, daidzein, resveratrol, fermented soy with a measured content of daidzein, and the invention composition (resveratrol + equol) (10 mol/l, for 24 hours) on the mRNA expression of mitochondrial biogenesis factors: nuclear respiratory factor (Nrf-1), mitochondrial transcription factor A (Tfam), and Peroxisome proliferator-activated receptor (PPAR)-α coactivator-1 (PGC-1 α) in HUVA using Light Cycler (Roche Molecular Biochemicals) as described in Scapagnini G, Butterfield DA, Colombrita C, Sultana R, Pascale A, Calabrese V. (2004) Ethyl ferulate, a lipophilic polyphenol, induces HO-1 and protects rat neurons against oxidative stress. Antioxid Redox Signal. 6(5):811-8.)

The total RNA was isolated with the isolation kit with Mini RNA (Zymo Research, Orange, CA) and was inverse-transcribed using Superscript III RT (Invitrogen) as described in Scapagnini G, D'Agata V, Calabrese V, Pascale A, Colombrita C, Alkon DL, Cavallaro S. (2002) Gene expression profiles of heme oxygenase isoforms in the rat brain Brain Res. 954(1): 31-39

The effectiveness of the PCR reaction was determined employing a series of standard sample dilutions. The housekeeping gene HPRT was used for internal normalization. The PCR reaction reproducibility was determined via melting point analysis and viewing the product on 2% agarose gel

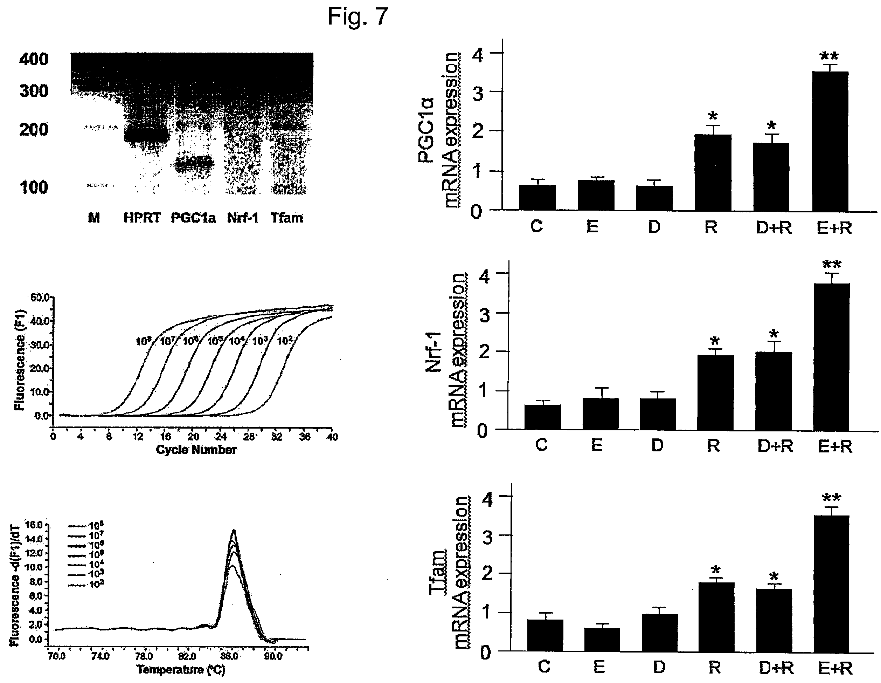

These results are summarized and illustrated in Figure 7

### EXAMPLE 5

### Quantification of the lipid content

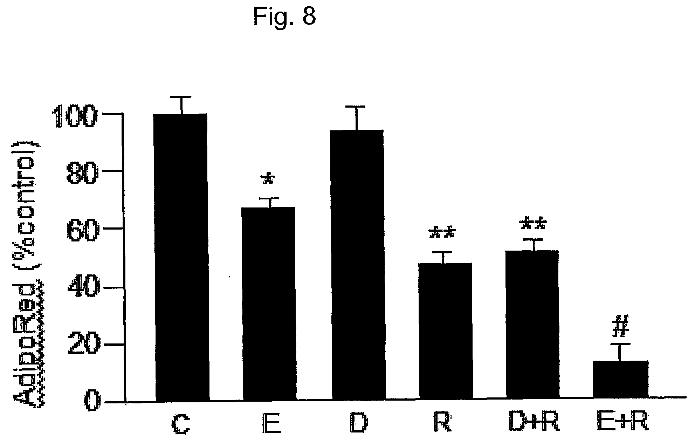

The lipid content was quantified using the commercially available assay reagent AdipoRed. In short, post-confluent preadipocytes were grown in 96-well plates and incubators with 0.2% DMSO or with equol, daidzein, resveratrol, fermented soy with a measured content of daidzein or the invention composition (resveratrol + equol) (10 mol/l, for 24 hours) during the adipogenic phase, and on the sixth day were assayed for lipid content in accordance with the instruction manual. The experiments were performed with at least 6 replicas per treatment and repeated 3 times. The results obtained are shown in Fig. 8.

### EXAMPLE 6

Table 4 demonstrates the absence of equol in I) a soy extract with a measured content of ginestein and daidzein, and in II) a supplement based on fermented soy with a measured content of aglyconic isoflavones.

A third sample analyzed is III) a product of a fermentation with conditioned bacteria capable of producing S-equol, that represents a source that can be used as a food supplement of this molecule.

The equol levels in the solutions to be tested were determined in triplicate via kits based on "Quantitative time-resolved fluoroimmunoassay" (Labmaster, Turku, Finland) technology.

In accordance with the recommended procedures, read via Victor 3 Multilabel Readers (PerkinElmer, Milan, Italy) and interpolated by means of Sigma Plot (Systat Software).

### RESULTS

The first two samples I) soy extract and II) fermented soy standard, exhibited values near the detectability limit of the method.

In sample III) with a soy base fermented with conditioned strains, it was possible to detect significant quantities of equol (approximately 8 mg/100 mg of fermented extract). **Table 4**

| Equol (nmol/L) | 1 mg |
|---|---|
| I) Dry soy extract with measured content of isoflavones | 10.5 ± 3 |
| II) Fermented soy with measured content of isoflavones | 14.6 ± 7 |
| III) Fermented soy with conditioned bacteria with a measured content of isoflavones | 1874.0 ± 2 |

### EXAMPLE 7

Formulation of an invention composition in tablet form for the treatment of menopause syndrome (sample dosage 1-3 tablets/day).

| **Description** | **Stated Active Ingredient** | **% Ex. Dos.** | **mg/un.** | **g/100 g** |
|---|---|---|---|---|
| Bulking agent: microcrystalline cellulose | | | 207.000 | 31.364 |
| **Dry extract of fermented soy (equol content: 20%)** | **20% isoflavones and total derivatives** | | **500.000** | 30.303 |
| Bulking agent: dicalcium phosphate | | | 150.000 | 22.727 |
| **Resveratrol from *Vitis vinifera*** | **98% in trans-resveratrol** | | **10.000** | 3.788 |
| Anti-caking agent: crosslinked sodium carboxy methyl cellulose | | | 16.000 | 2.424 |
| Glazing agent: hydroxypropyl methyl cellulose | | | 13.000 | 1.970 |
| Anti-caking agent: mono- and diglycerides of fatty acids | | | 12.000 | 1.818 |
| Stabilizing agent: polyvinylpyrrolidone | | | 10.000 | 1.515 |
| Anti-caking agent: magnesium stearate - of vegetable origin | | | 10.000 | 1.515 |
| Anti-caking agent: silicon dioxide | | | 10.000 | 1.515 |
| Colorant: titanium dioxide | | | 3.000 | 0.455 |
| Glazing agent: microcrystalline cellulose | | | 2.000 | 0.303 |
| Glazing agent: stearic acid | | | 2.000 | 0.303 |
| **Total** | | **Tot.** | 945.00 | 100.000 |

### EXAMPLE 8

Formulation of an invention composition for the treatment of the symptoms or disorders related to aging (dosage 1-3 tablets/day).

| **Description** | **Stated Active Ingredient** | **% Ex. Dos.** | **mg/un.** | **g/100 g** |
|---|---|---|---|---|
| Bulking agent: microcrystalline cellulose | | | 207.000 | 31.364 |
| **Dry extract of fermented soy Equol content 5%** | **60% isoflavones and total derivatives** | | **400.000** | 30.303 |
| Bulking agent: dicalcium phosphate | | | 150.000 | 22.727 |
| **Resveratrol from *Vitis vinifera*** | **30% monomers and oligomers** | | **50.000** | 3.788 |
| Anti-caking agent: crosslinked sodium carboxy methyl cellulose | | | 16.000 | 2.424 |
| Glazing agent: hydroxypropyl methyl cellulose | | | 13.000 | 1.970 |
| Anti-caking agent: mono- and diglycerides of fatty acids | | | 12.000 | 1.818 |
| Stabilizing agent: polyvinylpyrrolidone | | | 10.000 | 1.515 |
| Anti-caking agent: magnesium stearate - of vegetable origin | | | 10.000 | 1.515 |
| Anti-caking agent: silicon dioxide | | | 10.000 | 1.515 |
| Colorant: titanium dioxide | | | 3.000 | 0.455 |
| Glazing agent: microcrystalline cellulose | | | 2.000 | 0.303 |
| Glazing agent: stearic acid | | | 2.000 | 0.303 |
| **Total** | | **Tot.** | 885.00 | 100.000 |

### EXAMPLE 9

A randomized clinical study was conducted and the effectiveness of a food supplement on disorders and menopause events was controlled with placebos.
1.2. Nature and scope of the study. The study is intended to evaluate the effectiveness of a food supplement capable of preventing or treating the menopause syndrome.
For this purpose, a randomized clinical study, controlled with placebos, was conducted on 60 female subjects (30 subjects per line of treatment) of age between 50 and 55 years in menopause for less than one year.
In particular, it was studied whether or not the product was able to improve the symptoms accompanying the cessation of gonadal activity, such as: hot flushes, insomnia, and psychological disorders (anxiety, emotional instability, depression).
1.3. Product studied: Menopause supplement:
bulking agent: microcrystalline cellulose 207 mg,
equol originating from fermented soy (40% isoflavones and total derivatives) 200 mg,
bulking agent: dicalcium phosphate 150 mg,
Resveratrol from *Vitis vinifera* 25 mg,
anti-caking agent: crosslinked sodium carboxy methyl
cellulose 16 mg,
glazing agent: hydroxypropyl methyl cellulose 13 mg,
anti-caking agent: Glyceryl behenate 12 mg,
stabilizing agent: polyvinylpyrrolidone,
anti-caking agent: magnesium stearate - of vegetable origin,
anti-caking agent: silicon dioxide,
colorant: titanium dioxide,
glazing agent: microcrystalline cellulose,
glazing agent: stearic acid.
Menopause supplement (placebo):
   bulking agent: dicalcium phosphate 341 mg,
   bulking agent: microcrystalline cellulose 273 mg,
   glazing agent: hydroxypropyl methyl cellulose 10.4 mg,
   anti-caking agent: magnesium stearate - of vegetable origin 40 mg,
   anti-caking agent: silicon dioxide 40 mg,
   anti-caking agent: crosslinked sodium carboxy methylcellulose 6 mg,
   glazing agent: shellac 4 mg,
   colorant: E171 2.4 mg,
   glazing agent: microcrystalline cellulose 1.6 mg,
   glazing agent: stearic acid 1.6 mg.
1.4. Ethical requirements
   The study was approved by the "Independent Ethics Committee for Non Pharmacological Clinical Investigations" (on March 30, 2011, ref. 2011/02) and conducted in observance of the following ethical requirements.
   1.4.1. All subjects participating in the study are healthy volunteers at least 18 years of age.
   1.4.2. All the subjects participating in the study are selected under the supervision of a dermatologist with the application of inclusion/exclusion criteria (see § 1.5.1.1..-2.).
   1.4.3. All the subjects participating in the study are volunteers informed of the purpose and nature of the study.
   1.4.4. All the subjects participating in the study were informed of any risk entailed in the performance of the study.
   1.4.5. All the subjects participating in the study gave their informed consent signing a document prior to the start of the study.
   1.4.6. Before the volunteers are exposed to the product under study, all the health and safety information relevant to the product and its individual components are evaluated.
   1.4.7. All the study procedures are conducted in conformity with the ethical principles for research in the medical field (Ethical principles for medical research that involves human subjects, adopted by the 18th General Assembly of the WMA at Helsinki, Finland, in June 1964) and subsequent amendments.
   1.4.8 All the precautions necessary to prevent the occurrence of adverse cutaneous reactions were taken.
   1.4.9. Should there be unexpected/adverse reactions, the experimenter physician judges the severity thereof
      (reporting it in detail) and accordingly implements appropriate care.

5. Subjects participating in the study
1.5.1. Selecting the subjects
   The subjects participating in the study are selected from a panel of healthy female subjects (between 50 and 55 years of age, inclusive) applying the inclusion and exclusion criteria reported below.
   1.5.1.1. Inclusion criteria
      Healthy female subjects
      Age: between 50 and 55 years of age, inclusive
      Race: Caucasian
      Body mass index between 20 and 25, inclusive
      Slightly overweight (10-20% with respect to the ideal weight)
      Onset of menopause (date of the last menstrual cycle) no longer than 1 year with respect to date of enrollment in the study
      Anamnestic clinical picture characterized by psychological disorders (anxiety, emotional instability, depression) typical of the menopause syndrome, hot flushes, localized accumulations of fat, increase of mass
      Subjects who have not participated in similar studies
      Absence of pathologies
      Commitment to not change the normal daily routine
      Commitment to not use products with activity that could superimpose that of the product under study for the entire period of the study
      Commitment to not change eating habits
      Subjects knowledgeable of the study who have signed an informed consent document
   1.5.1.2. Exclusion criteria
      Subjects that do not satisfy the inclusion criteria
      Subjects in both local and systemic pharmacological therapy
      Subjects whose medical history do not exhibit past episodes of endometrial hyperplasia
      Hormone replacement therapy
      Metabolic syndrome
      Depressive syndrome not correlated with menopause
      Subjects with food intolerances

1.6. MATERIALS AND METHODS
1.6.1. Conducting the study
1.6.1.1 PHASE I - Selecting the subjects
In this phase, 60 female subjects are recruited from a panel of healthy subjects in menopause for less than one year. The experimenter evaluates: the general state of health of the subject and her eligibility to participate in the study (compliance with the inclusion criteria/exclusion).
1.6.1.2 Phase II - Start and end of the study
After enrollment, the subjects are subdivided into the following experimental groups:
Group A: subjects (n =30) who take the active ingredient belong to this group.
Group B: subjects (n =30) who take the placebo belong to this group.

For each volunteer, the experimenter evaluates the basal conditions (T0). Afterwards, the subjects are instructed how to use the product, and they are given the factsheet of the study and the product under discussion.
Subsequent controls at 1 (T1) and 3 (T3) months of treatment are then scheduled. 1.6.2. Ratings
1.6.2.1. Product effectiveness in improving psychological disorders (anxiety, emotional instability, depression) and the hot flushes that accompany the menopause syndrome.
The following parameters are monitored before the study and at a distance of 1 and 3 months of treatment:
- Via the Hamilton Depression Rating scale (Hamilton M.A rating scale for depression. J Neurol Neurosurg Psychiatry 1960; 23:56-62) the improvement of the mood intensity is rated (reduction of the symptoms correlated with the depression). The questionnaire given to the volunteers is shown in Table 5.
- The change in sleep disorders is rated via the "Nottingham Health profile" rating scale. The questionnaire given to the volunteers is shown in Table 6.
- The change in the symptoms associated with menopause is evaluated via the "Menopause Rating Scale, MRS" (Lothar AJ Heinemann, Peter Potthoff, and HermanPG Schneider. International versions of the Menopause Rating Scale (MRS). Health Qual Life Outcomes. 2003;1:28). The questionnaire given to the volunteers is shown in Table 7.

**Table 5 Hamilton M.M. rating scale for depression**

| **Symptoms** | **Intensity** |
|---|---|
| Hot flushes, sweating (episodes of sweating) | None -Mild -Moderate -Severe -Very severe |
| Heart discomfort (unusual awareness of heart beat, heart skipping, heart racing, tightness) | None -Mild -Moderate -Severe -Very severe |
| Sleep problems (difficulty in falling asleep, difficulty in sleeping through, waking up early) | None -Mild -Moderate -Severe -Very severe |
| Depressive mood (feeling down, sad, on the verge of tears, lack of drive, mood swings) | None -Mild -Moderate -Severe -Very severe |
| Irritability (feeling nervous, inner tension, feeling aggressive) | None -Mild -Moderate -Severe -Very severe |
| Physical and mental exhaustion (general decrease in performance, impaired memory, decrease in concentration, forgetfulness) | None -Mild -Moderate -Severe -Very severe |
| Sexual problems (change in sexual desire, in sexual activity and satisfaction) | None -Mild -Moderate -Severe -Very severe |
| Bladder problems (difficulty in urinating, increased need to urinate, bladder incontinence) Dryness of vagina (sensation of dryness or burning in the vagina, difficulty with sexual intercourse) | None -Mild -Moderate -Severe -Very severe |
| Dryness of vagina (sensation of dryness or burning in the vagina, difficulty with sexual intercourse) | None -Mild -Moderate -Severe -Very severe |
| Joint and muscular discomfort (pain in the joints, rheumatoid complaints) | None -Mild -Moderate -Severe -Very severe |

**Table 6 Hamilton M.M. rating scale for depression**

| | |
|---|---|
| Work and activities | 0 No difficulty -1 Thoughts and feelings of unability, fatigue or weakness related to activities; work or hobbies -2 Loss of interest in activity; hobbies or work -3 Decrease in actual time spent in activities or decrease in productivity -4 Stopped working because of present illness |
| Agitation | 0 None 1 Fidgetiness -2 Playing with hands, hair, etc. -3 Moving about, can't sit still -4 Hand wringing, nail biking, hair polling, biking of lips |
| Anxiety (psycological) | 0 No difficulty -1 Subjective tension and irritabilità -2 Worryng about minor matters -3 Apprehensive attitude apparent in face or speech -4 Fears expressed without questioning |
| Anxiety somatic. Physiological concomitants of anxiety (i.e., effects of autonomic overactivity, "butterflies", indigestion, stomach cramps, belching, diarrhea, palpitations, hyperventilation, paresthesia, sweating, flushing, tremor, headache, urinary frequency). | 0 Absent -1 Mild -2 Moderate -3 Severe -4 Incapacitating |

**Table 7 Nottingham Health profile**

| **Symptoms** | **Intensity** |
|---|---|
| I take pills to help me sleep | YES -NO |
| I'm waking up in the early hours of the morning | YES -NO |
| I lie awake for most of the night | YES -NO |
| It takes me a long time to get to sleep | YES -NO |
| I sleep badly at night | YES -NO |

1.6.2.2. Results
The results of the questionnaires are expressed in percentage values and shown in the respective tables.
"MENOPAUSE RATING SCALE, MRS" FOR SYMPTOMS ASSOCIATED WITH MENOPAUSE
(Lothar AJ Heinemann, Peter Potthoff, and HermanPG Schneider. International versions of the Menopause Rating Scale (MRS). Health Qual. Life Outcomes. 2003;1:28)
The severity of the symptoms associated with menopause was assessed by means of the "Menopause Rating Scale, MRS." Upon enrollment, the panel is composed as described in table 8.

**Table 8 Panel composition at T0**

| **ACTIVE GROUP** | | | |
|---|---|---|---|
| **Symptoms** | **Score** | **Grading** | **% of Subjects** |
| Hot flushes, sweating | 3.2 ± 0.2 | Moderate | 100.0% |
| Heart discomfort | 1.8 ± 0.1 | Mild | 63.3% |
| Sleep problems | 3.1 ± 0.1 | Moderate | 100.0% |
| Depressive mood | 2.0 ± 0.1 | Mild | 66.7% |
| Irritability | 2.5 ± 0.2 | Mild | 76.7% |
| Physical and mental exhaustion | 2.5 ± 0.1 | Moderate | 86.7% |
| Sexual problems | 2.8 ± 0.1 | Moderate | 100.0% |
| Bladder problems | 2.1 ± 0.2 | Mild | 66.7% |
| Dryness of vagina | 2.7 ± 0.1 | Moderate | 93.3% |
| Joint and muscular discomfort | 3.0 ± 0.2 | Moderate | 90.0% |

| **PLACEBO GROUP** | | | |
|---|---|---|---|
| **Symptoms** | **Score** | **Grading** | **% of Subjects** |
| Hot flushes, sweating | 3.1 ± 0.1 | Moderate | 100.0% |
| Heart discomfort | 1.9 ± 0.1 | Mild | 66.7% |
| Sleep problems | 3.0 ± 0.2 | Moderate | 100.0% |
| Depressive mood | 2.0 ± 0.1 | Mild | 66.7% |
| Irritability | 2.5 ± 0.2 | Mild | 83.3% |
| Physical and mental exhaustion | 2.4 ± 0.1 | Moderate | 83.3% |
| Sexual problems | 2.8 ± 0.1 | Moderate | 100.0% |
| Bladder problems | 2.1 ± 0.1 | Mild | 83.3% |
| Dryness of vagina | 2.6 ± 0.1 | Moderate | 93.3% |
| Joint and muscular discomfort | 2.9 ± 0.2 | Moderate | 93.3% |

The two groups of subjects (Active and Placebo) are homogeneous with reference to the initial symptoms anticipated by MRS. Statistical analysis (Mann-Whitney U test) does not reveal significant differences (p>0.05) in the medians.

"MENOPAUSE RATING SCALE, MRS" FOR SYMPTOMS ASSOCIATED WITH MENOPAUSE

(Lothar AJ Heinemann, Peter Potthoff, and HermanPG Schneider. International versions of the Menopause Rating Scale (MRS). Health Qual. Life Outcomes. 2003; 1:28)

Table 8 shows the evolution of menopausal symptomatology 1 to 3 months after taking the active product and the placebo product. The data show the percentage of subjects that exhibit an improvement* in the clinical symptomatology.

**Table 8a - Variation of the menopausal symptoms**

| **ACTIVE GROUP** | | |
|---|---|---|
| **Symptoms** | **T1** | **T3** |
| Hot flushes, sweating | 53.3% | 73.3% |
| Heart discomfort | 63.2% | 78.9% |
| Sleep problems | 36.7% | 53.3% |
| Depressive mood | 0.0% | 75.0% |
| Irritability | 43.5% | 60.9% |
| Physical and mental exhaustion | 26.9% | 65.4% |
| Sexual problems | 23.3% | 73.3% |
| Bladder problems | 50.0% | 80.0% |
| Dryness of vagina | 17.9% | 85.7% |
| Joint and muscular discomfort | 66.7% | 81.5% |

| **PLACEBO GROUP** | | |
|---|---|---|
| **Symptoms** | **T1** | **T3** |
| Hot flushes, sweating | 23.3% | 20.0% |
| Heart discomfort | 35.0% | 45.0% |
| Sleep problems | 33.3% | 16.7% |
| Depressive mood | 0.0% | 35.0% |
| Irritability | 32.0% | 32.0% |
| Physical and mental exhaustion | 4.0% | 28.0% |
| Sexual problems | 20.0% | 3.3% |
| Bladder problems | 8.0% | 24.0% |
| Dryness of vagina | 3.6% | 3.6% |
| Joint and muscular discomfort | 21.4% | 21.4% |

| | | |
|---|---|---|
| * the percentage of subjects that exhibit an improvement is calculated relative to number of subjects who demonstrate a degree of symptomatology greater than "absent" at the start (subjects reporting the symptoms to be absent are excluded from the calculation). | | |

"MENOPAUSE RATING SCALE, MRS" FOR SYMPTOMS ASSOCIATED WITH MENOPAUSE (Lothar AJ Heinemann, Peter Potjthoff, and HermanPG Schneider. International versions of the Menopause Rating Scale (MRS). Health Qual. Life Outcomes. 2003;1:28)

Table 8b shows the intra- and intergroup statistical analysis. The intragroup statistical analysis is performed via the Wilcoxon test of the ranges indicated, whereas the intergroup analysis is performed via the Mann-Whitney U test.

**Table 8b Statistical analysis**

| **ACTIVE GROUP** | | |
|---|---|---|
| **Symptoms** | **T1** | **T3** |
| Hot flushes, sweating | ** | *** |
| Heart discomfort | *** | *** |
| Sleep problems | *** | *** |
| Depressive mood | n.s. | *** |
| Irritability | ** | *** |
| Physical and mental exhaustion | * | *** |
| Sexual problems | n.s. | *** |
| Bladder problems | * | *** |
| Dryness of vagina | n.s. | *** |
| Joint and muscular discomfort | *** | *** |

| **PLACEBO GROUP** | | |
|---|---|---|
| **Symptoms** | **T1** | **T3** |
| Hot flushes, sweating | n.s. | n.s. |
| Heart discomfort | n.s. | n.s. |
| Sleep problems | n.s. | n.s. |
| Depressive mood | n.s. | ** |
| Irritability | ** | * |
| Physical and mental exhaustion | n.s. | ** |
| Sexual problems | * | n.s. |
| Bladder problems | n.s. | * |
| Dryness of vagina | n.s. | n.s. |
| Joint and muscular discomfort | * | * |

| **INTERGROUP ANALYSIS** | | |
|---|---|---|
| **Symptoms** | **T1** | **T3** |
| Hot flushes, sweating | * | *** |
| Heart discomfort | ** | * |
| Sleep problems | n.s. | ** |
| Depressive mood | n.s. | * |
| Irritability | n.s. | * |
| Physical and mental exhaustion | *** | ** |
| Sexual problems | n.s. | *** |
| Bladder problems | n.s. | ** |
| Dryness of vagina | n.s. | *** |
| Joint and muscular discomfort | ** | *** |

| | | |
|---|---|---|
| n.s. not significant - *, p<0.05 - **, p<0.01 - ***, p<0.001 | | |

HAMILTON DEPRESSION RATING SCALE

(Hamilton M.A rating scale for depression. J Neurol Neurosurg Psychiatry 1960; 23:56-62)

The mood intensity was evaluated by means of the "Hamilton Depression Rating Scale" Upon enrollment, the panel is composed as described in table 2.

**Table 9 Panel composition at T0**

| **ACTIVE GROUP** | | | |
|---|---|---|---|
| **Symptoms** | **Score** | **Grading** | **% of Subjects** |
| Work and activities | 2.1 ± 0.2 | Loss of interest in activity | 56.7% |
| Agitation | 1.4 ± 0.1 | Fidgetiness | 70.0% |
| Anxiety (psycological) | 1.4 ± 0.2 | Subjective tension and irritability | 63.3% |
| Anxiety somatic | 1.8 ± 0.2 | Moderate | 63.3% |

| **PLACEBO GROUP** | | | |
|---|---|---|---|
| **Symptoms** | **Score** | **Grading** | **% of Subjects** |
| Work and activities | 2.0 ± 0.1 | Loss of interest in activity | 60.0% |
| Agitation | 1.5 ± 0.1 | Fidgetiness | 66.7% |
| Anxiety (psycological) | 1.4 ± 0.1 | Subjective tension and irritability | 63.3% |
| Anxiety somatic | 1.7 ± 0.1 | Moderate | 60.0% |

The two groups of subjects (Active and Placebo) are homogeneous with reference to the initial symptoms anticipated by the Hamilton scale. The statistical analysis (Mann-Whitney U test) does not indicate significant differences (p>0.05) in the medians.

HAMILTON DEPRESSION RATING SCALE

(Hamilton M.A rating scale for depression. J Neurol Neurosurg Psychiatry 1960; 23:56-62)

Table 9 shows the evolution of the mood intensity 1 to 3 months after taking the active product and the placebo product. The data show the percentage of subjects that exhibit an improvement* in the clinical symptomatology.

**Table 9a Variation of the mod**

| **ACTIVE GROUP** | | |
|---|---|---|
| **Symptoms** | **T1** | **T3** |
| Work and activities | 76.5% | 94.1% |
| Agitation | 23.8% | 52.4% |
| Anxiety (psycological) | 31.6% | 68.4% |
| Anxiety somatic | 31.6% | 63.2% |

| **PLACEBO GROUP** | | |
|---|---|---|
| **Symptoms** | **T1** | **T3** |
| Work and activities | 33.3% | 38.9% |
| Agitation | 19.0% | 28.6% |
| Anxiety (psycological) | 15.0% | 20.0% |
| Anxiety somatic | 19.0% | 23.8% |

| | | |
|---|---|---|
| * the percentage of subjects that exhibit an improvement is calculated relative to number of subjects who demonstrate a degree of symptomatology greater than "absent" at the start (subjects reporting the symptoms to be absent are excluded from the calculation). | | |

HAMILTON DEPRESSION RATING SCALE

(Hamilton M.A rating scale for depression. J Neurol Neurosurg Psychiatry 1960; 23:56-62)

Table 9b shows the intra- and intergroup statistical analysis. The intragroup statistical analysis is performed via the Wilcoxon test of the ranges indicated, whereas the intergroup analysis is performed via the Mann-Whitney U test.

**Table 9b Statistical analysis**

| **ACTIVE GROUP** | | |
|---|---|---|
| **Symptoms** | **T1** | **T3** |
| Work and activities | ** | *** |
| Agitation | n.s. | ** |
| Anxiety (psycological) | n.s. | *** |
| Anxiety somatic | n.s. | *** |

| **PLACEBO GROUP** | | |
|---|---|---|
| **Symptoms** | **T1** | **T3** |
| Work and activities | * | ** |
| Agitation | n.s. | n.s. |
| Anxiety (psycological) | n.s. | n.s. |
| Anxiety somatic | n.s. | * |

| **INTERGROUP ANALYSIS** | | |
|---|---|---|
| **Symptoms** | **T1** | **T3** |
| Work and activities | * | *** |
| Agitation | n.s. | * |
| Anxiety (psycological) | n.s. | ** |
| Anxiety somatic | n.s. | * |

| | | |
|---|---|---|
| n.s. not significant - *, p<0.05 - **, p<0.01 - ***, p<0.001 | | |

"NOTTINGHAM HEALTH PROFILE" SLEEP DISORDER RATING SCALE

The severity of the sleep disorders associated with menopause was evaluated by means of the "Nottingham Health Profile."

Upon enrollment, the panel is composed as described in table 10.

**Table 10 - Panel composition at TO**

| **ACTIVE GROUP** | |
|---|---|
| **Symptoms** | **% of Subjects** |
| I take pills to help me sleep | 43.3% |
| I'm waking up in the early hours of the morning | 63.3% |
| I lie awake for most of the night | 20.0% |
| It takes me a long time to get to sleep | 40.0% |
| I sleep badly at night | 63.3% |

| **PLACEBO GROUP** | |
|---|---|
| **Symptoms** | **% of Subjects** |
| I take pills to help me sleep | 40.0% |
| I'm waking up in the early hours of the morning | 60.0% |
| I lie awake for most of the night | 23.3% |
| It takes me a long time to get to sleep | 40% |
| I sleep badly at night | 60% |

The two groups of subjects (Active and Placebo) are homogeneous with reference to the initial symptoms anticipated by the MRS. The statistical analysis (Mann-Whitney U test) does not indicate significant differences (p>0.05) in the medians.

"NOTTINGHAM HEALTH PROFILE" SLEEP DISORDER RATING SCALE

Table 10 shows the evolution of the sleep disorders 1 to 3 months after taking the active product and the placebo product. The data show the percentage of subjects that exhibit an improvement* in the clinical symptomatology.

**Table 10a - Variation of the sleep disorders**

| **ACTIVE GROUP** | | |
|---|---|---|
| **Symptoms** | **T1** | **T3** |
| I take pills to help me sleep | 7.7% | 46.2% |
| I'm waking up in the early hours of the morning | 21.1% | 52.6% |
| I lie awake for most of the night | 16.7% | 83.3% |
| It takes me a long time to get to sleep | 8.3% | 58.3% |
| I sleep badly at night | 36.8% | 63.2% |

| **PLACEBO GROUP** | | |
|---|---|---|
| **Symptoms** | **T1** | **T3** |
| I take pills to help me sleep | 0.0% | 8.3% |
| I'm waking up in the early hours of the morning | 0.0% | 11.1% |
| I lie awake for most of the night | 14.3% | 14.3% |
| It takes me a long time to get to sleep | 8.3% | 16.7% |
| I sleep badly at night | 5.6% | 22.2% |

| | | |
|---|---|---|
| * the percentage of subjects that exhibit an improvement is calculated relative to number of subjects who demonstrate a degree of symptomatology at the start (subjects reporting the symptoms to be absent are excluded from the calculation). | | |

"NOTTINGHAM HEALTH PROFILE" SLEEP DISORDER RATING SCALE

Table 10b shows the intra- and intergroup statistical analysis. The intragroup statistical analysis is performed via the Wilcoxon test of the ranges indicated, whereas the intergroup analysis is performed via the Mann-Whitney U test.

**Table 10b - Statistical analysis**

| **ACTIVE GROUP** | | |
|---|---|---|
| **Symptoms** | **T1** | **T3** |
| I take pills to help me sleep | n.s. | * |
| I'm waking up in the early hours of the morning | n.s. | ** |
| I lie awake for most of the night | * | * |
| It takes me a long time to get to sleep | n.s. | ** |
| I sleep badly at night | ** | *** |

| **PLACEBO GROUP** | | |
|---|---|---|
| **Symptoms** | **T1** | **T3** |
| I take pills to help me sleep | n.s. | n.s. |
| I'm waking up in the early hours of the morning | n.s. | n.s. |
| I lie awake for most of the night | n.s. | n.s. |
| It takes me a long time to get to sleep | n.s. | n.s. |
| I sleep badly at night | n.s. | n.s. |

| **INTERGROUP ANALYSIS** | | |
|---|---|---|
| **Symptoms** | **T1** | **T3** |
| I take pills to help me sleep | n.s. | * |
| I'm waking up in the early hours of the morning | * | ** |
| I lie awake for most of the night | n.s. | * |
| It takes me a long time to get to sleep | n.s. | * |
| I sleep badly at night | * | * |

| | | |
|---|---|---|
| n.s. not significant - *, p<0.05 - **, p<0.01 - ***, p<0.001 | | |

### CONCLUSIONS

The ingestion (1 tablet/day) of the product (food supplement) previously identified results in an improvement of the clinical symptomatology of menopause. The product's effect can already be seen after the first month of use. This effect is superior to that recorded for the placebo product, as shown by the statistical analysis reported in the table below:

**Table 11**

| **Scale** | **Symptoms** | T1 | T3 |
|---|---|---|---|
| MRS | Hot flushes, sweating | * | *** |
| MRS | Heart discomfort | ** | * |
| MRS | Sleep problems | n.s. | ** |
| MRS | Depressive mood | n.s. | * |
| MRS | Irritability | n.s. | * |
| MRS | Physical and mental exhaustion | ** * | ** |
| MRS | Sexual problems | n.s. | *** |
| MRS | Bladder problems | n.s. | ** |
| MRS | Dryness of vagina | n.s. | *** |
| MRS | Joint and muscular discomfort | ** | *** |
| HA | Work and activities | * | *** |
| HA | Agitation | n.s. | * |
| HA | Anxiety (psycological) | n.s. | ** |
| HA | Anxiety somatic | n.s. | * |
| NHP | I take pills to help me sleep | n.s. | * |
| NHP | I'm waking up in the early hours of the morning | * | ** |
| NHP | I lie awake for most of the night | n.s. | * |
| NHP | It takes me a long time to get to sleep | n.s. | * |
| NHP | I sleep badly at night | * | * |
| not significant-*, p<0.05-**, p<0.01-***, p<0.001 MRS, Menopause rating scale -HA, Hamilton scale -NHP, Notthingam health profile. | | | |

### DEMOGRAPHY

The table below shows the panel characteristics

**Table 12**

| | **Menopausal food supplement Batch FH226 (Equopausa Tablets)** | **Menopausal food supplement Batch FH314 (Equopausa placebo)** |
|---|---|---|
| Sample size | 30 subjects | 30 subjects |
| Sex | Female | Female |
| BMI | 20<BMI<25 | 20<BMI<25 |
| Overweight | ≥10e \| and ≤20 | ≥10e \| and ≤20 |
| Menopause onset | < 1 year from enrolment date | < 1 year from enrolment date |

**Table 13 - AVERAGE DATA**

| The data are reported as average +/- SEM | | | | | | |
|---|---|---|---|---|---|---|
| MRS - MENOPAUSE RATING SCALE | 0 MONTHS | | 1MONTHS | | 3 MONTHS | |
| | ACTIVE | PLACEBO | ACTIVE | PLACEBO | ACTIVE | PLACEBO |
| 1- Hot flushes, sweating (episodes of sweating) | 3,2 ± 0,2 | 3,1 ± 0,1 | 2,8 ± 0,2 | 3,1 ± 0,1 | 2,3 ± 0,2 | 3,0 ± 0,1 |
| 2- Heart discomfort (unusual awareness of heart beat, heart skipping, heart racing, tightness) | 1,8 ± 0,1 | 1,9 ± 0,1 | 1,2 ± 0,1 | 1,9 ± 0,1 | 1,1 ± 0,1 | 1,7 ± 0,1 |
| 3- Sleep problems Sleep problems (difficulty in falling asleep, difficulty in sleeping through, waking up early) | 3,1 ± 0,1 | 3,0 ± 0,2 | 2,8 ± 0,2 | 2,8 ± 0,1 | 2,6 ± 0,1 | 2,9 ± 0,2 |
| 4- Depressive mood (feeling down, sad, on the verge of tears, lack of drive, mood swings) | 2,0 ± 0,1 | 2,0 ± 0,1 | 2,0 ± 0,1 | 2,0 ± 0,2 | 1,4 ± 0,1 | 1,7 ± 0,2 |
| 5- Irritability Irritability (feeling nervous, inner tension, feeling aggressive) | 2,5 ± 0,2 | 2,5 ± 0,2 | 2,1 ± 0,2 | 2,2 ± 0,2 | 1,9 ± 0,2 | 2,3 ± 0,2 |
| 6- Physical and mental exhaustion (general decrease in performance, impaired memory, decrease in concentration, forgetfulness) | 2,5 ± 0,1 | 2,4 ± 0,1 | 2,2 ± 0,2 | 2,8 ± 0,1 | 1,8 ± 0,1 | 2,3 ± 0,2 |
| 7-Sexual problems Sexual problems (change in sexual desire, in sexual activity and satisfaction) | 2,8 ± 0,1 | 2,8 ± 0,1 | 2,7 ± 0,2 | 2,6 ± 0,1 | 2,0 ± 0,1 | 3,0 ± 0,1 |
| 8- Bladder problems Bladder problems (difficulty in urinating, increased need to urinate, bladder incontinence) | 2,1 ± 0,2 | 2,1 ± 0,1 | 1,8 ± 0,2 | 2,0 ± 0,1 | 1,4 ± 0,1 | 1,9 ± 0,1 |
| 9- Dryness of vagina Dryness of vagina (sensation of dryness or burning in the vagina, difficulty with sexual intercourse) | 2,7 ± 0,1 | 2,6 ± 0,1 | 2,5 ± 0,1 | 2,6 ± 0,1 | 1,9 ± 0,1 | 2,6 ± 0,1 |
| 10- Joint and muscular discomfort (pain in the joints, rheumatoid complaints) | 3,0 ± 0,2 | 2,9 ± 0,2 | 2,3 ± 0,1 | 2,7 ± 0,2 | 2,2 ± 0,2 | 2,7 ± 0,2 |

**Table 14 - Average data**

| The data are reported as % average of the SI. | | | | | | |
|---|---|---|---|---|---|---|
| NHP - NOTTINGHAM HEALTH PROFILE Subarea "sleep" | 0 MONTHS | | 1 MONTHS | | 3 MONTHS | |
| | ACTIVE | PLACEBO | ACTIVE | PLACEBO | ACTIVE | PLACEBO |
| 1 I take pills to help me sleep | 43,3% | 40,0% | 40,0% | 40,0% | 23,3% | 36,7% |
| 2 I'm waking up in the early hours of the morning | 63,3% | 60,0% | 50,0% | 60,0% | 30,0% | 53,3% |
| 3 I lie away for most of the night | 20,0% | 23,3% | 16,7% | 23,3% | 3,3% | 20,0% |
| 4 It takes me a long time to get to sleep | 40,0% | 40,0% | 36,7% | 36,7% | 16,7% | 33,3% |
| 5 I sleep badly at night | 63,3% | 60,0% | 40,0% | 56,7% | 23,3% | 46,7% |

**Table 15 - Average data**

| The data are reported as average +/- SEM | | | | | | |
|---|---|---|---|---|---|---|
| HAMILTON DEPRESSION SCALE | 0 MONTHS | | 1 MONTHS | | 3 MONTHS | |
| | ACTIVE | PLACEBO | ACTIVE | PLACEBO | ACTIVE | PLACEBO |
| 1 Work and activities | 2,1 ± 0,2 | 2,0 ± 0,1 | 0,8 ± 0,1 | 1,6 ± 0,1 | 0,5 ± 0,1 | 1, 5 ± 0,1 |
| 2 Agitation | 1,4 ± 0,1 | 1,5 ± 0,1 | 1,2 ± 0,2 | 1,4 ± 0,1 | 0,7 ± 0,2 | 1,5 ± 0,2 |
| 3 Anxiety (psycological) | 1,6 ± 0,2 | 1,4 ± 0,1 | 1,4 ± 0,2 | 1,3 ± 0,1 | 0,9 ± 0,2 | 1,3 ± 0,1 |
| 4 Anxiety somatic | 1,8 ± 0,2 | 1,7 ± 0,1 | 1,3 ± 0,1 | 1,6 ± 0,1 | 0,7 ± 0,1 | 1,3 ± 0,1 |

## Claims

1. A composition for use in the prevention and/or the treatment of menopausal syndrome comprising equol and resveratrol in effective quantities, at least one edible carrier, and/or excipient.

2. Composition for use in accordance with claim 1, containing equol in an amount ranging from 1 to 100 mg.

3. Composition for use in accordance with claim 1 wherein equol is contained in a fermented-soy extract having a titre from 20 to 60% in total isoflavones

4. Composition in accordance with claim 1 wherein the equol is in the form of S-equol.

5. Composition for use in accordance with claim 1, wherein the resveratrol is contained in a *Vitis vinifera* extract.

6. Composition for use in accordance with claim 5, wherein the *Vitis vinifera* extract comprises 1.25-5 mg of resveratrol.

7. Composition for use in accordance with claim 1, wherein the composition comprises a *Vitis vinifera* extract in a quantity of 10-200 mg.

8. Composition for use in accordance with claim 1 wherein said menopausal syndrome includes one or more affections selected from depression, insomnia, anxiety, cardiovascular disorders, osteoporosis, urinary ailments, pelvic pains, imbalances of blood lipid fractions.

9. Composition for use in accordance with any one of the claims 1-8 in the form of a nutraceutical preparation or food supplement.

10. The non-therapeutic use of a composition in the form of a nutraceutical preparation or food supplement comprising equol and resveratrol in effective quantities, at least one edible carrier, and/or excipient for reducing the menstrual syndrome disorders selected from hot flushes, dryness of the skin, redness of the skin, perspirationand combinations thereof.

11. The non-therapeutic use of a composition in the form of a nutraceutical preparation or food supplement comprising equol and resveratrol in effective quantities, at least one edible carrier, and/or excipient for reducing skin aging.

## Patentansprüche

1. Zusammensetzung zur Verwendung bei der Vorbeugung und/oder Behandlung des Menopausensyndroms, umfassend Equol und Resveratrol in wirksamen Mengen, mindestens einen essbaren Träger und/oder einen Hilfsstoff.

2. Zusammensetzung zur Verwendung gemäß Anspruch 1, enthaltend Equol in einer Menge im Bereich von 1 bis 100 mg.

3. Zusammensetzung zur Verwendung gemäß Anspruch 1, wobei Equol in einem fermentierten Soja-Extrakt mit einem Titer von 20 bis 60 % Gesamt-Isoflavonen vorhanden ist.

4. Zusammensetzung gemäß Anspruch 1, wobei Equol in der Form von S-Equol vorliegt.

5. Zusammensetzung zur Verwendung gemäß Anspruch 1, wobei Resveratrol in einem Vitis-vinifera-Extrakt enthalten ist.

6. Zusammensetzung zur Verwendung gemäß Anspruch 5, wobei das Vitis-vinifera-Extrakt 1,25 - 5 mg Resveratrol enthält.

7. Zusammensetzung zur Verwendung gemäß Anspruch 1, wobei die Zusammensetzung einen Vitis-vinifera-Extrakt in einer Menge von 10 - 200 mg enthält.

8. Zusammensetzung zur Verwendung gemäß Anspruch 1, wobei genanntes Menopausensyndrom ein oder mehrere Erkrankungen umfasst, die aus Depression, Schlaflosigkeit, Angst, kardiovaskulären Störungen, Osteoporose, Beschwerden des Harntrakts, Beckenschmerzen oder Ungleichgewichten der Blutfettanteile ausgewählt sind.

9. Zusammensetzung zur Verwendung gemäß einem der vorhergehenden Ansprüche 1 bis 8 in der Form eines nutrazeutischen Präparats oder Nahrungsergänzungsmittels.

10. Nicht therapeutische Verwendung einer Zusammensetzung in der Form eines nutrazeutischen Präparats oder Nahrungsergänzungsmittels, umfassend Equol und Resveratrol in wirksamen Mengen, mindestens einen essbaren Träger und/oder Hilfsstoff zur Verringerung von Menstruationsbeschwerden, ausgewählt aus Hitzewallungen, trockener Haut, Schwitzen und Kombinationen davon.

11. Nicht therapeutische Verwendung einer Zusammensetzung in der Form eines nutrazeutischen Präparats oder Nahrungsergänzungsmittels, umfassend Equol und Resveratrol in wirksamen Mengen, mindestens einen essbaren Träger und/oder Hilfsstoff zur Verringerung der Hautalterung.

## Revendications

1. Composition à utiliser pour la prévention et/ou le traitement de syndrome de la ménopause et/ou du vieillissement, comprenant de l'équol et du resvératrol en des quantités efficaces, au moins un support comestible et/ou un excipient.

2. Composition à utiliser selon la revendication 1, contenant de l'équol en une quantité allant de 1 à 100 mg.

3. Composition à utiliser selon la revendication 1, dans laquelle l'équol est contenu dans un extrait de soja fermenté, ayant un titre d'isoflavones de 20 à 60 % au total.

4. Composition selon la revendication 1, dans laquelle l'équol est sous la forme de S-équol.

5. Composition à utiliser selon la revendication 1, dans laquelle le resvératrol est contenu dans un extrait de *Vitis vinifera.*

6. Composition à utiliser selon la revendication 5, dans laquelle l'extrait de *Vitis vinifera* comprend de 1,25 à 5 mg de resvératrol.

7. Composition à utiliser selon la revendication 1, dans laquelle la composition comprend un extrait de *Vitis vinifera* en une quantité de 10 à 200 mg.

8. Composition à utiliser selon la revendication 1, dans laquelle ledit syndrome de la ménopause comporte une ou plusieurs affections choisies parmi la dépression, l'insomnie, l'anxiété, les troubles cardiovasculaires, l'ostéoporose, les maladies des voies urinaires, les douleurs pelviennes, des déséquilibres de fractions de lipides sanguins.

9. Composition à utiliser selon l'une quelconque des revendications 1 à 8, sous la forme d'une préparation nutraceutique ou d'un complément alimentaire.

10. Utilisation non-thérapeutique d'une composition sous la forme d'une préparation nutraceutique ou d'un complément alimentaire, comprenant de l'équol et du resvératrol en des quantités efficaces, au moins un support comestible et/ou un excipient pour réduire les troubles du syndrome prémenstruel choisis parmi les bouffées de chaleur, une sécheresse de la peau, des rougeurs de la peau, la transpiration et des combinaisons de celles-ci-ci.

11. Utilisation non-thérapeutique d'une composition sous la forme d'une préparation nutraceutique ou d'un complément alimentaire, comprenant de l'équol et du resvératrol en des quantités efficaces, au moins un support comestible et/ou un excipient, pour réduire le vieillissement de la peau.
